Europäisches Patentamt

European Patent Office

Office européen des brevets

(11) Veröffentlichungsnummer: **0 304 713**
**A1**

## EUROPÄISCHE PATENTANMELDUNG

(21) Anmeldenummer: **88112852.4**

(22) Anmeldetag: **06.08.88**

(51) Int. Cl.⁴: **A61K 7/06 , A45D 34/00**

(30) Priorität: **27.08.87 DE 3728550**

(43) Veröffentlichungstag der Anmeldung:
**01.03.89 Patentblatt 89/09**

(84) Benannte Vertragsstaaten:
**AT BE CH DE ES FR GB IT LI NL SE**

(71) Anmelder: **Wella Aktiengesellschaft**
**Berliner Allee 65**
**D-6100 Darmstadt(DE)**

(72) Erfinder: **Abels, Willi**
**85 Ross Avenue**
**Demarest New Jersey 07627(US)**
Erfinder: **Mager, Herbert, Dr.**
**Beaumont 5**
**CH-1700 Fribourg(CH)**
Erfinder: **Aeby, Johann**
**Rte. Câtelet 5**
**CH-1723 Marly(CH)**

(54) **Sprühbares Haar-oder Körperreinigungsmittel und Verfahren zur schonenden Reinigung der Haare oder des Körpers.**

(57) Wäßriges, von haarverformenden Wirkstoffen und Treibgasen freies, mit einer mechanischen Sprühvorrichtung versprühbares Haar- und Körperreinigungsmittel, welches eine Viskosität von 0,001 bis 30 mPa·s bei 30 Grad Celsius aufweist und

    A) 3 - 30 Gewichtsprozent eines amphoteren Tensids aus der Klasse der Betaine,

    B) 0,01 - 1,0 Gewichtsprozent eines kationischen Cellulosederivats und

    C) 0,01 - 6 Gewichtsprozent 1,2-Propylenglykol enthält sowie ein Verfahren zum Reinigen der Haare und des Körpers. Das erfindungsgemäße Mittel zeichnet sich durch ein sehr gutes Reinigungsvermögen und eine hervorragende physiologische Verträglichkeit aus, während das neue Verfahren einen sparsamen Verbrauch des Mittels und eine sehr gute Schonung der Haare und der Haut gewährleistet.

**Sprühbares Haar- oder Körperreinigungsmittel und Verfahren zur schonenden Reinigung der Haare oder des Körpers**

Gegenstand der Erfindung ist ein haut- und schleimhautschonendes Haar- oder Körperreinigungsmittel mit sehr niedriger Viskosität sowie ein Verfahren zur Reinigung der Haare oder des Körpers.

Übliche Haar- oder Körperreinigungsmittel enthalten als waschaktive Substanzen meist anionische Tenside und weisen in der Regel eine Viskosität von 1000 bis 3000 mPa*s bei 30 Grad Celsius auf, welche durch den Zusatz von verdickend wirkenden Substanzen, wie zum Beispiel anorganischen Salzen oder Alkylolamiden, eingestellt wird.

Dickflüssige Shampoos werden vom Verbraucher bevorzugt, da er im allgemeinen eine hohe Viskosität mit einem hohen Wirkstoffgehalt gleichsetzt.

Als anionische Tenside kommen vor allem Alkylsulfate und Alkylethersulfate in Betracht. Während die Alkylsulfate in einem nicht unerheblichen Maße haut- und schleimhautreizend wirken, sind die in dermatologischer Hinsicht etwas günstigeren Alkylethersulfate in jüngster Zeit wegen ihres Gehalts an Dioxan negativ beurteilt worden.

In speziellen Haar- oder Körperreinigungsmitteln werden amphotere Tenside, wie zum Beispiel Fettsäureamidoalkylbetaine, eingesetzt, welche neben einem guten Schaum- und Reinigungsvermögen eine ausgezeichnete Haut- und Schleimhautverträglichkeit besitzen. Wäßrige Lösungen von amphoteren Tensiden aus der Klasse der Betaine lassen sich durch die Zugabe von anorganischen Salzen nicht verdicken. Als verdickend wirkende Substanzen werden hier Alkylolamide eingesetzt, die jedoch zu einer unerwünschten starken Rückfettung der Haare führen. Dadurch werden die vorzüglichen Eigenschaften dieser amphoteren Tenside in der Praxis der Haar- oder Körperreinigung wieder beeinträchtigt.

Die bekannten dickflüssigen Haar- oder Körperreinigungsmittel werden üblicherweise in einem Behälter in den Handel gebracht, der gleichzeitig als Auftrageflasche dient. Bei dem üblichen Verfahren zur Reinigung der Haare und des Körpers wird aus der Auftrageflasche eine für die Reinigung ausreichende Menge des dickflüssigen Mittels in das Zentrum der zu reinigenden Körperstelle oder auf das Haar gebracht und anschließend durch Reiben über die gesamte zu reinigende Stelle verteilt. Dies hat den Nachteil, daß zunächst die Gesamtmenge des aufgetragenen Mittels an einer Stelle konzentriert ist und dort infolge der hohen Wirkstoffkonzentration unerwünschte Nebenwirkungen hervorrufen kann. Weiterhin ist ein zweiter Arbeitsgang notwendig, um das Mittel über die gesamte zu reinigende Oberfläche der Haare oder der Haut zu verteilen.

Es sind auch Aerosol-Shampoos bekannt, bei denen die reinigend wirkende Zusammensetzung mit Hilfe eines Treibgases aus dem Behälter, üblicherweise in Form eines Schaumes, auf die zu reinigenden Stellen aufgetragen wird. Die Reinigungsmittel müssen dann ebenfalls noch über die gesamte zu reinigende Fläche verteilt werden. Darüberhinaus werden manche Treibgase, insbesondere Fluorchlorkohlenwasserstoffe, im Hinblick auf mögliche Umweltschäden, sowie Propan und Butan, infolge ihrer Brennbarkeit, negativ beurteilt.

Aus der US-PS 4 243 659 ist eine wäßrige, haarverformende Shampoozusammensetzung bekannt, welche neben Dimethylharnstoff und Natriumhydrogensulfit ein amphoteres Betain-Tensid, kationische Cellulose sowie Propylenglykol enthält. Der US-PS 4 243 659 liegt jedoch eine andere Zielsetzung als der vorliegenden Anmeldung zugrunde, nämlich das Haar während des Waschens gleichzeitig umzuformen. Dementsprechend ist die dort beschriebene Zusammensetzung durch die darin enthaltenen haarverformenden oder haarquellenden Wirkstoffe haut- und schleimhautreizend.

Es bestand daher die Aufgabe, ein Haar- oder Körperreinigungsmittel zur Verfügung zu stellen, welches eine ausgezeichnete Haut- und Schleimhautverträglichkeit aufweist, ein gutes Reinigungs- und Schaumvermögen besitzt, weiterhin besser dosierbar und verteilbar als übliche derartige Mittel sowie frei von Treibgasen ist.

Es wurde nun gefunden, daß durch ein wäßriges, von haarverformenden Wirkstoffen und Treibgasen freies, mit einer mechanischen Sprühvorrichtung versprühbares Haar- oder Körperreinigungsmittel, dadurch gekennzeichnet, daß es eine Viskosität von 0,001 bis 30 mPa*s bei 30 Grad Celsius aufweist und

A) 3 - 30 Gewichtsprozent eines amphoteren Tensids aus der Klasse der Betaine,

B) 0,01 - 1,0 Gewichtsprozent eines kationischen Cellulosederivats und

C) 0,01 - 6 Gewichtsprozent 1,2-Propylenglykol enthält, die vorstehende Aufgabe in hervorragender Weise gelöst wird.

Die Viskosität des erfindungsgemäßen Mittels beträgt vorzugsweise 0,01 bis 10 mPa*s bei 30 Grad Celsius.

Ganz besonders bevorzugt sind solche erfindungsgemäßen Haar- oder Körperreinigungsmittel, deren Viskosität 0,5 bis 10 mPa·s bei 30 Grad Celsius, gemessen mit einer Haake-Viskowaage, Stab II und einem Auflagegewicht von 5 g, beträgt

Als amphoteres Tensid aus der Klasse der Betaine, werden vorzugsweise $(C_6-C_{22})$-Fettsäureamidoalkyl-betaine, beispielsweise $(C_6-C_{22})$-Fettsäureamidopropyl-dimethylamino-essigsäurebetain, oder $(C_6-C_{22})$-Alkylbetaine, beispielsweise Kokosfettalkyl-dimethylamino-essigsäurebetain, vorzugsweise in einer Menge von etwa 5 bis 20 Gewichtsprozent eingesetzt.

Kokosfettalkyl steht hierbei für ein Gemisch aus gesättigten $(C_6-C_{20})$- und ungesättigten $C_{18}$-Alkylresten, dessen Zusammensetzung den im Kokosfett enthaltenen Fettsäureresten entspricht.

Als kationisches Cellulosederivat kommen insbesondere solche Verbindungen in Betracht, wie sie in der US-PS 3 472 840 beschrieben sind und im Handel unter der Bezeichnung Polymer® JR erhältlich sind, wobei kationische Cellulosederivate, deren 2prozentige wäßrige Lösungen bei 25 Grad Celsius eine Viskosität von etwa 75 - 175 mPa·s, gemessen mit einem Brookfield LVF-Rotationsviskosimeter bei einer Spindelgeschwindigkeit von 30 Umdrehungen pro Minute, aufweisen, bevorzugt sind. Die bevorzugte Einsatzmenge des kationischen Cellulosederivats beträgt 0,05 bis 0,8 Gewichtsprozent.

Selbstverständlich können in dem erfindungsgemäßen Haar- oder Körperreinigungsmittel bekannte, für derartige Mittel übliche kosmetische Zusatzstoffe enthalten sein, sofern die hervorragende Haut- und Schleimhautverträglichkeit nicht beeinträchtigt wird und die niedrige Viskosität erhalten bleibt.

Als geeignete kosmetische Zusatzstoffe können beispielsweise anionische, kationische, amphotere oder nichtionische Netzmittel und Emulgatoren, wie zum Beispiel $C_{12}-C_{18}$-Alkylethersulfate, Alkyltrimethylammoniumsalze, Alkylpyridiniumsalze, Carboxyderivate von Imidazol, N-Alkylsulfobetaine oder Polyglycerylether von gesättigten oder ungesättigten Fettalkoholen und Alkylphenolen in einer Menge von etwa 0,01 bis 3 Gewichtsprozent, ferner Konservierungsstoffe, wie zum Beispiel Salicylsäure oder Mandelsäure in einer Menge von etwa 0,01 bis 0,7 Gewichtsprozent, sowie Antischuppenwirkstoffe, wie zum Beispiel Zink-Pyridinthion, kosmetische Farbstoffe, wie zum Beispiel Fluorescein-Natriumsalz, weiterhin haar- und hautpflegende Wirkstoffe, wie zum Beispiel Fettsäureester, Fettalkohole, Fettsäureglyceride, Chitosanderivate, Lanolinderivate oder Pantothensäure in einer Menge von etwa 0,01 bis 3 Gewichtsprozent, sowie ferner Komplexbildner, Schaumstabilisatoren, Puffersubstanzen, Lichtschutzmittel oder Parfümöle in einer Menge von etwa 0,01 - 0,8 Gewichtsprozent enthalten sein.

Der Wassergehalt des erfindungsgemäßen Mittels liegt vorzugsweise zwischen 75 und 90 Gewichtsprozent, besonders bevorzugt zwischen 80 und 86 Gewichtsprozent.

Das neue Mittel besitzt ein gutes Schaum- und Reinigungsvermögen sowie eine ausgezeichnete Haut- und Schleimhautverträglichkeit.

Ein weiterer Vorteil des erfindungsgemäßen Mittels ist durch den Gehalt an einem kationischen Cellulosederivat gegeben, wodurch insbesondere auf trockenem oder geschädigtem Haar eine konditionierende Wirkung erzielt wird.

Als wesentliche Voraussetzung für die Funktionsfähigkeit eines sprühbaren Haar- und Körperreinigungsmittels ist die niedrige Viskosität zu nennen, die auch während der Lagerung nicht ansteigen darf. Bei einem Ansteigen der Viskosität, würde der Behälterinhalt nicht mehr versprüht, sondern verspritzt werden, wodurch ein gleichmäßiges Auftragen auf die zu reinigende Stelle nicht mehr gewährleistet ist. 1,2-Propylenglykol hat sich als besonders geeignet erwiesen, um die Viskosität auf dem gewünschten niedrigen Niveau zu halten. Darüberhinaus wird durch 1,2-Propylenglykol das Schaumvermögen der Betaine nicht oder nur in sehr geringem Maße beeinflußt. Die bevorzugte Einsatzmenge des 1,2-Propylenglykols beträgt 1 bis 5 Gewichtsprozent.

Gegenüber bekannten verdickten Haar- oder Körperreinigungsmitteln ist das neue Mittel besser dosier- und verteilbar. Dies führt zu einem sparsameren Verbrauch und zu einer schonenderen Behandlung der Haare und der Haut.

Ein weiterer Vorteil des erfindungsgemäßen Mittels besteht darin, daß es frei von Treibgasen ist, wodurch die nachteiligen Eigenschaften der mit Treibgas betriebenen Sprays, wie zum Beispiel mögliche Umweltschäden oder erhöhte Brennbarkeit, vermieden werden.

Die Anwendung des neuen Haar- oder Körperreinigungsmittels mittels einer mechanischen Sprühvorrichtung, hat den Vorteil, daß der Verpackungsinhalt weitgehend gegen eine Verkeimung von außen geschützt ist. Deshalb kann die Konservierung durch Zusatz einer geringeren Menge des Konservierungsstoffs erreicht werden, was wiederum der Verträglichkeit zugute kommt.

Unter mechanischen Sprühvorrichtungen sind solche Vorrichtungen zu verstehen, welche das Versprühen einer Flüssigkeit ohne Verwendung eines Treibgases ermöglichen.

Gegenstand der vorliegenden Erfindung ist weiterhin ein Verfahren zur haut- und schleimhautschonenden Reinigung des Körpers oder der Haare, dadurch gekennzeichnet, daß man eine für die Reinigung ausreichende Menge, welche von der Größe der zu reinigenden Körperfläche beziehungsweise von der Fülle der Haare abhängt und etwa 2 bis 15 g beträgt, des vorstehend beschriebenen erfindungsgemäßen Mittels aus einem mit einer mechanischen Sprühvorrichtung versehenen Behälter, ohne Verwendung eines Treibgases, fein verteilt auf die Haut oder die Haare aufsprüht, sodann die Haut oder die Haare shampooniert, wobei die Einwirkungszeit des Mittels etwa 10 Sekunden bis 15 Minuten dauert, und danach das Mittel mit Wasser abspült.

Als geeignete mechanische Sprühvorrichtung kann beispielsweise eine Sprühpumpe oder ein mit einem Sprühventil versehener elastischer Behälter, in den das vorstehend beschriebene Haar- oder Körperreinigungsmittel unter Druck abgefüllt wird, wobei sich der elastische Behälter ausdehnt und aus dem sich das Mittel infolge der Kontraktion des elastischen Behälters beim Öffnen des Sprühventils kontinuierlich entnehmen läßt, verwendet werden. Sprühvorrichtungen der zuletzt genannten Art sind beispielsweise in den Patentschriften US-PS 3 672 543, US-PS 3 738 538, US-PS 3 791 557, US-PS 3 796 356, US-PS 3 876 115, US-PS 3 961 725 und EP-PS 0 058 700 beschrieben und werden zum Beispiel unter der Bezeichnung "Exxel"-Behälter von der Firma Container Industries, New Jersey, USA vertrieben.

Durch das vorstehend beschriebene Verfahren ist gewährleistet, daß nicht wie bei üblichen Verfahren zur Reinigung der Haare und der Haut, das Mittel in hoher Konzentration auf eine kleine Fläche der zu reinigenden Stelle aufgetragen wird, was zu einer Auslaugung der Haut führen kann. Weiterhin ist das Mittel nach dem Aufsprühen bereits über die gesamte zu reinigende Fläche verteilt, wodurch der Arbeitsgang der Verteilung, wie er bei üblichen derartigen Mitteln notwendig ist, entfällt.

Die folgenden Beispiele sollen den Gegenstand der Erfindung näher erläutern, ohne ihn darauf zu beschränken.

## Beispiele

### Beispiel 1 Sprühshampoo mit einer Viskosität von 7 mPa·s

| | |
|---|---|
| 40,0 g | Kokosfettsäureamidopropyl-dimethylamino-essigsäure-betain, 30prozentige wäßrige Lösung |
| 0,5 g | kationische Cellulose, deren 2prozentige wäßrige Lösung bei 25 Grad Celsius eine Viskosität von 75 - 175 mPa·s aufweist |
| 3,0 g | 1,2-Propylenglykol |
| 0,2 g | Konservierungsstoff |
| 0,3 g | Parfümöl |
| 56,0 g | Wasser |
| 100,0 g | |

Das Mittel nach Beispiel 1 wird mittels Luftdruck durch das Ventil eines "Exxel"-Behälters abgefüllt. Durch Öffnen des Ventils werden 5 g des Mittels auf das Haar aufgesprüht. Das Versprühen ist kontinuierlich ohne Pumpen möglich. Durch geeignete handelsübliche Sprühköpfe kann ein kegelförmiges Sprühbild erzielt werden. Nach dem Aufsprühen des Mittels wird das Haar shampooniert und das Mittel nach einer Einwirkungszeit von 1 Minute mit Wasser ausgespült. Das Haar wird schonend und gründlich gereinigt.

### Beispiel 2 Sprühshampoo mit einer Viskosität von 2 mPa·s

| | |
|---|---|
| 40,0 g | Kokosfettalkyl-dimethylamino-essigsäure-betain, 32prozentige wäßrige Lösung |
| 0,1 g | kationische Cellulose, deren 2prozentige wäßrige Lösung bei 25 Grad Celsius eine Viskosität von 75 - 175 mPa•s besitzt |
| 2,0 g | 1,2-Propylenglykol |
| 0,2 g | Konservierungsstoff |
| 0,3 g | Parfümöl |
| 57,4 g | Wasser |
| 100,0 g | |

Das Mittel nach Beispiel 2 wird in einen für kosmetische Mittel üblichen Kunststoffbehälter abgefüllt, der mit einer handelsüblichen Sprühpumpe ausgestattet ist. Durch Betätigen der Pumpe werden 8 g des Mittels auf das Haar aufgesprüht, sodann das Haar shampooniert, und nach einer Einwirkungszeit von 2 Minuten wird das Mittel mit Wasser ausgespült. Das Haar wird sehr schonend und gründlich gereinigt.

**Beispiel 3 Körperreinigungsmittel mit einer Viskosität von 5 mPa•s**

| | |
|---|---|
| 50,00 g | Kokosfettsäureamidopropyl-dimethylamino-essigsäure-betain, 30prozentige wäßrige Lösung |
| 0,25 g | kationische Cellulose, deren 2prozentige wäßrige Lösung bei 25 Grad Celsius eine Viskosität von 75 - 175 mPa•s besitzt |
| 3,00 g | 1,2-Propylenglykol |
| 0,20 g | Konservierungsstoff |
| 0,30 g | Parfümöl |
| 46,25 g | Wasser |
| 100,00 g | |

Das Körperreinigungsmittel nach Beispiel 3 wird in einen für kosmetische Mittel üblichen Kunststoffbehälter abgefüllt, der mit einer handelsüblichen Sprühpumpe ausgestattet ist. Durch Betätigen der Pumpe werden 2 g des Mittels auf die zu reinigende Stelle des Körpers aufgesprüht, sodann die Haut shampooniert und nach einer Einwirkungszeit von 30 Sekunden das Mittel mit Wasser abgespült. Die so behandelte Körperstelle wird hautschonend und sehr gründlich gereinigt.

Alle Prozentangaben dieser Anmeldung stellen, sofern nicht anders angegeben, Gewichtsprozente dar. Die Viskositäten wurden, sofern nicht anders angegeben, mit einer Haake-Viskowaage bei 30 Grad Celsius, Stab II und einem Auflagegewicht von 5 g bestimmt.

**Ansprüche**

1. Wäßriges, von haarverformenden Wirkstoffen und Treibgasen freies, mit einer mechanischen Sprühvorrichtung versprühbares Haar- oder Körperreinigungsmittel, dadurch gekennzeichnet, daß es eine Viskosität von 0,001 bis 30 mPa•s bei 30 Grad Celsius aufweist und
A) 3 - 30 Gewichtsprozent eines amphoteren Tensids aus der Klasse der Betaine,
B) 0,01 - 1,0 Gewichtsprozent eines kationischen Cellulosederivats und
C) 0,01 - 6 Gewichtsprozent 1,2-Propylenglykol
enthält.

2. Mittel nach Anspruch 1, dadurch gekennzeichnet, daß es eine Viskosität von 0,01 bis 10 mPa•s bei 30 Grad Celsius aufweist.

3. Mittel nach Anspruch 1, dadurch gekennzeichnet, daß es eine Viskosität von 0,5 bis 10 mPa•s bei 30 Grad Celsius aufweist.

4. Mittel nach einem der Ansprüche 1 bis 3, dadurch gekennzeichnet, daß das amphotere Tensid ein $(C_6\text{-}C_{22})$-Fettsäureamidoalkylbetain ist.

5. Mittel nach einem der Ansprüche 1 bis 4, dadurch gekennzeichnet, daß das amphotere Tensid ein $(C_6\text{-}C_{22})$-Alkylbetain ist.

5. Mittel nach einem der Ansprüche 1 bis 4, dadurch gekennzeichnet, daß das kationische Cellulosederivat in 2prozentiger wäßriger Losung eine Viskosität von 75 - 175 mPa•s bei 25 Grad Celsius besitzt.

6. Mittel nach einem der Ansprüche 1 bis 6, dadurch gekennzeichnet, daß es Parfümöle, Konservierungsstoffe, anionische, kationische, amphotere oder nichtionische Netzmittel und Emulgatoren, Komplexbildner, Lichtschutzmittel, Antischuppenwirkstoffe, kosmetische Farbstoffe, Schaumstabilisatoren, Puffersubstanzen sowie haar- und hautpflegende Wirkstoffe enthält.

7. Verfahren zur Reinigung des Körpers oder der Haare, dadurch gekennzeichnet, daß man eine für die Reinigung ausreichende Menge des Mittels nach einem der Ansprüche 1 bis 6 aus einem mit einer mechanischen Sprühvorrichtung versehenen Behälter, ohne Verwendung eines Treibgases, fein verteilt auf die Haut oder die Haare aufsprüht, sodann die Haut oder die Haare shampooniert und das Mittel nach einer Einwirkungszeit von 10 Sekunden bis 15 Minuten mit Wasser abspült.

8. Verfahren nach Anspruch 7, dadurch gekennzeichnet, daß die mechanische Sprühvorrichtung eine Sprühpumpe ist.

9. Verfahren nach Anspruch 7, dadurch gekennzeichnet, daß die mechanische Sprühvorrichtung ein mit einem Sprühventil versehener elastischer Behälter ist, in den das vorstehend beschriebene Haar- oder Körperreinigungsmittel unter Druck abgefüllt wird, wobei sich der elastische Behälter ausdehnt und aus dem sich das Mittel infolge der Kontraktion des elastischen Behälters beim Öffnen des Sprühventils kontinuierlich entnehmen läßt.

**Europäisches Patentamt**

# EUROPÄISCHER RECHERCHENBERICHT

Nummer der Anmeldung

EP 88 11 2852

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 117 135 (JOHNSON & JOHNSON) * Ansprüche 1,3,7-9,11 * --- | 1,4,5 | A 61 K 7/06 A 45 D 34/00 |
| Y | DE-A-3 302 456 (HENKEL) * Anspruch 6; Seite 7, Zeilen 21-34, insbesondere Zeile 33; Seite 8, Zeilen 1-5, insbesondere Zeile 3; Seite 9, Zeilen 1-6 * --- | 1,4,5 | |
| D,Y | US-A-4 243 659 (A.T. BALINGIT) * Ansprüche 1,4-6,11,12 * --- | 1,4,5 | |
| A | DE-A-3 604 811 (L. BUSSMEIER) * Ansprüche * ----- | 7-9 | |
| | | | **RECHERCHIERTE SACHGEBIETE (Int. Cl.4)** |
| | | | A 61 K A 45 D |

Der vorliegende Recherchenbericht wurde für alle Patentansprüche erstellt

| Recherchenort | Abschlußdatum der Recherche | Prüfer |
|---|---|---|
| DEN HAAG | 14-11-1988 | SCARPONI U. |